# EUROPEAN PATENT APPLICATION

(11) **EP 3 366 208 A1**
(43) Date of publication of application: **29.08.2018**
(21) Application number: 17158054.1
(22) Date of filing: 27.02.2017
(51) Int. Cl.: A61B 5/053, A61B 5/11, A61B 5/00

(54) **A DEVICE AND METHOD FOR MONITORING A USER**

(71) Applicant: Koninklijke Philips N.V., 5656 AE Eindhoven (NL)
(72) Inventor: SHUIJERS, Erik Gosuinus Petrus, 5656 AE Eindhoven (NL); COX, Lieke, 5656 AE Eindhoven (NL)
(74) Representative: de Haan, Poul Erik

(57) **Abstract**

There is provided a device (100) and method of operating the device for monitoring a user. The device (100) comprises a processor (102) configured to detect a daily routine of the user of the device, a detector (104) configured to monitor one or more physiological characteristics of the user, and a user interface (106) configured to indicate day-based information about the one or more monitored physiological characteristics of the user. A start time point for the day-based information is determined based on the detected daily routine of the user.

## Description

### Technical Field of the Invention

The invention relates to the field of monitoring of a user and, in particular, to a device and a method of operating the device for monitoring the user.

### Background to the Invention

In many situations, it can be beneficial to monitor a user of a device. For example, by monitoring a user of a device, it is possible to gain an insight into the lifestyle and the health of the user. Often, the devices that are used to monitor a user can provide instantaneous, real-time feedback to the user on lifestyle or health parameters. Examples of such parameters include heart rate, energy expenditure, number of steps, number of active minutes, number of sedentary minutes, and so on. The feedback that is provided by devices used to monitor a user can be indicative of parameters at a current time (for example, the current heart rate of the user) or can be a cumulative measure of the parameters for the current day (for example, the amount of steps taken by the user over an entire day).

The parameters acquired by monitoring a user can be particularly useful in coaching programs. In these coaching programs, it is often daily metrics that are used as input. For example, the feedback given to a user can be based specifically around daily metrics, such as an instruction to the user to increase the amount of daily steps to a certain value or to increase the amount of daily active minutes to a certain value. However, the use of daily metrics requires knowledge of what constitutes "a day".

Typically, the concept of "a day" is implemented by resetting daily parameter values at midnight. For a user that goes to bed before midnight (such as at 23:00), this does not pose a problem. However, for a user with a shifted day/night rhythm that goes to bed after midnight (such as at 03:00), the perception of "a day" is different. For example, where the parameter is the number of steps, any steps that a user may have taken after midnight (for example, from midnight until 03:00) will already be present when the user wakes up. However, the perception of the user is that the day only starts when they wake up. This problem is even more apparent for shift workers. For example, for a user that wakes up at 20:00 and subsequently starts a working day, it is inconvenient for the parameters to be reset to zero at midnight. This may be counteracted by providing the user with the control to reset the daily parameters. However, this can be a tedious action for the user, which is also prone to error.

There exist methods that aim at linking physiological measurements of a user to the circadian rhythm of the user. An example of such a method is provided in WO 2015/189107 A1. However, even with these methods, the user is not provided with information that is relevant to a day as the user actually perceives it.

There is thus a need for an improved device and method of operating the device for monitoring a user.

### Summary of the Invention

As noted above, the limitation with existing approaches is that a user is not provided with feedback associated with a day as the user actually perceives it. It would thus be valuable to have an improved device and method of operating the device for monitoring a user, which overcomes these existing problems.

Therefore, according to a first aspect of the invention, there is provided a device for monitoring a user. The device comprises a processor configured to detect a daily routine of the user of the device, a detector configured to monitor one or more physiological characteristics of the user, and a user interface configured to indicate day-based information about the one or more monitored physiological characteristics of the user. A start time point for the day-based information is determined based on the detected daily routine of the user.

In some embodiments, the processor may be configured to detect the daily routine of the user of the device by detecting a period of sleep of the user of the device. In some embodiments, the processor may be configured to detect a period of sleep of the user of the device exceeding a threshold duration. In some embodiments, the threshold duration may be in the range of 2-4 hours. In some embodiments, the processor may be configured to exclude short periods of wakefulness when detecting the period of sleep. In some embodiments, the end of the detected period of sleep may be taken as the start time point for the day-based information.

In some embodiments, the device may further comprise any one or more of: a motion sensor, wherein the processor may be configured to detect the period of sleep of the user of the device from an output of the motion sensor; and a heart rate sensor, wherein the processor may be configured to detect the period of sleep of the user of the device from an output of the heart rate sensor.

In some embodiments, the processor may be configured to detect a period of sleep of the user based on a probability that the user is asleep or awake.

In some embodiments, the processor is configured to detect the daily routine of the user of the device by detecting any one or more of: one or more activities performed by the user of the device; and an interaction of the user with the device or another device. In some embodiments, an occurrence of one or more activities associated with waking may be taken as the start time point for the day-based information. In some embodiments, the device may further comprise a receiver configured to receive an input from at least one separate device to detect the one or more activities performed by the user of the device.

In some embodiments, the detector may comprise any one or more of: a motion sensor; a heart rate sensor; a respiratory rate sensor; a skin response sensor; a muscle activity sensor; and a temperature sensor.

In some embodiments, the user interface may be configured to indicate the day-based information about the one or more monitored physiological characteristics of the user, normalised to a 24 hour period.

According to a second aspect of the invention, there is provided a method for monitoring a user. The method comprises detecting a daily routine of the user of the device, monitoring one or more physiological characteristics of the user, and indicating day-based information about the one or more monitored physiological characteristics of the user. A start time point for the day-based information is determined based on the detected daily routine of the user.

According to a third aspect of the invention, there is provided a computer program product comprising a computer readable medium, the computer readable medium having computer readable code embodied therein, the computer readable code being configured such that, on execution by a suitable computer or processor, the computer or processor is caused to perform the method or the methods described above.

According to the aspects and embodiments described above, the limitations of existing techniques are addressed. In particular, according to the above-described aspects and embodiments, it is possible to align the concept of "a day" with that of the user. This is possible through the use of sensor data to establish the daily routine of the user. There is thus provided an improved device and method of operating the device for monitoring a user, which overcomes the existing problems.

### Brief Description of the Drawings

For a better understanding of the invention, and to show more clearly how it may be carried into effect, reference will now be made, by way of example only, to the accompanying drawings, in which:
Figure 1 is a block diagram of a device according to an embodiment;
Figure 2 is a flow chart illustrating a method according to an embodiment;
Figure 3 an example illustration of time intervals for day-based information determined according to an embodiment; and
Figure 4 is a flow chart illustrating a method according to an example embodiment.

### Detailed Description of the Preferred Embodiments

As noted above, the invention provides an improved device and method of operating the device for monitoring a user, which overcomes the existing problems.

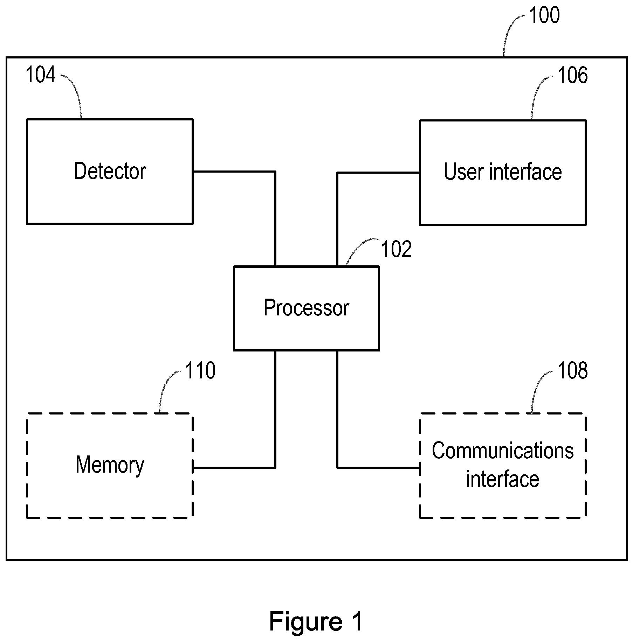

**Figure 1** shows a block diagram of a device 100 that can be used for monitoring a user according to an embodiment.

With reference to Figure 1, the device 100 comprises a processor 102 that controls the operation of the device 100 and that can implement the method described herein. In some embodiments, the device 100 can be a wearable device configured to be worn by the user (such as a smart watch, or any other wearable device). In other embodiments, the device 100 can be a mobile device (such as a smart phone, a tablet, a laptop computer, or any other mobile device).

The processor 102 of the device 100 can comprise one or more processing units, multicore processors or modules that are configured or programmed to control the device 100 in the manner described herein. In particular implementations, the processor 102 can comprise a plurality of software and/or hardware modules that are each configured to perform, or are for performing, individual or multiple steps of the method according to embodiments of the invention. The processor 102 of the device 100 is configured to detect a daily routine (or daily pattern) of the user of the device 100. The daily routine of the user of the device 100 may be detected automatically.

In some embodiments, the processor 102 may be configured to detect the daily routine of the user of the device 100 by detecting a period of sleep of the user of the device 100. In these embodiments, the device 100 can further comprise one or more detectors 104 configured to acquire information indicative of the period of sleep of the user and the processor 102 can be configured to detect the period of sleep of the user from an output of the one or more detectors 104. For example, the device 100 can further comprise a motion sensor (such as an accelerometer) and the processor 102 may be configured to detect the period of sleep of the user of the device 100 from an output of the motion sensor. Alternatively or in addition, the device 100 can further comprise a heart rate sensor and the processor 102 may be configured to detect the period of sleep of the user of the device 100 from an output of the heart rate sensor. In some embodiments, instead of or in addition to one or more detectors 104 configured to acquire information indicative of the period of sleep of the user, the processor 102 may be configured to acquire information indicative of the period of sleep of the user from at least one separate device.

In other embodiments, alternatively or in addition to detecting the daily routine based on a period of sleep of the user, the processor 102 may be configured to detect the daily routine of the user of the device 100 by detecting one or more activities performed by the user of the device 100. Examples of activities that may be detected include, but are not limited to, movement patterns of the user, time of meals of the user, or any other activities of the user, or any combination of activities of the user.

Alternatively or in addition, in some embodiments, the processor 102 may be configured to detect the daily routine of the user of the device 100 by detecting an interaction of the user with the device 100 itself or another device. The device with which the user is detected to interact can be any device with which the user can interact. For example, the device with which the user is detected to interact can be a device that the user first interacts with at the start of their day and last interacts with at the end of their day. Examples of the device with which the user is detected to interact include, but are not limited to, a mobile device (such as a mobile phone, a smartphone, or a tablet), a lighting device (such as a lamp, or a room mounted light), or any other device with which the user can interact. The device with which the user is detected to interact can comprise a sensor configured to detect a user interaction.

The device 100 also comprises a detector 104 configured to monitor one or more physiological characteristics of the user. The detector 104 configured to monitor one or more physiological characteristics of the user may comprise any one or more of a motion sensor, a heart rate sensor, a respiratory rate sensor, a skin response sensor, a muscle activity sensor, a temperature sensor (or, more specifically, a skin temperature sensor), or any other detector, or any combination of detectors, suitable to monitor one or more physiological characteristics of the user. In embodiments where the device 100 comprises one or more detectors configured to acquire information indicative of the period of sleep of the user for use in detecting the daily routine of the user (as described earlier), these one or more detectors may be different detectors, or at least some of the same detectors, as the detector that is configured to monitor one or more physiological characteristics of the user.

A motion (or activity or inertial) sensor may be any type of sensor, or any combination of sensors, suitable to acquire motion signals (such as acceleration signals, or any other motion signals) from the user. In some embodiments, the motion signals may be indicative of a sleep pattern of the user, a step count for the user, an energy expenditure of a user, a value for the active minutes of the user, a value for the sedentary minutes of the user, or any other motion information of the user. Examples of a motion sensor include, but are not limited to, an accelerometer, a gyroscope, a magnetometer, a passive infrared sensor (PIR) sensor, a camera, or any other motion sensor, or any combination of motion sensors.

A heart rate sensor may be any type of sensor, or any combination of sensors, suitable to acquire heart rate signals from the user. Examples of a heart rate sensor include, but are not limited to, an electrocardiogram (ECG) sensor, a photoplethysmography (PPG) sensor, a phonocardiography (PCG) sensor, a camera, or any other heart rate sensor, or any other heart rate sensor, or any combination of heart rate sensors.

A respiratory rate sensor may be any type of sensor, or any combination of sensors, suitable to acquire respiratory rate signals from the user. Examples of a respiratory rate sensor include, but are not limited to, a respiratory belt, an electrocardiogram (ECG) sensor, a photoplethysmography (PPG) sensor, a camera (for example, directed at the chest of the user), a motion sensor such as an accelerometer (for example, worn on the chest of the user), or any other respiratory rate sensor, or any combination of respiratory rate sensors.

A skin response sensor may be any type of sensor, or any combination of sensors, suitable to acquire skin response signals (such as skin conductivity signals) from the user. Examples of a skin response sensor include, but are not limited to, a galvanic skin response sensor, an optical sensor (such as a camera, or any other optical sensor), or any other skin response sensor, or any combination of skin response sensors.

A muscle activity sensor may be any type of sensor, or any combination of sensors, suitable to acquire muscle activity signals from the user. Examples of a muscle activity sensor include, but are not limited to, an electromyography (EMG) sensor, or any other muscle activity sensor, or any combination of muscle activity sensors.

A temperature sensor may be any type of sensor, or any combination of sensors, suitable to acquire temperature signals from the user. Examples of a temperature sensor include, but are not limited to, a thermometer, a thermistor, a thermocouple, a fibre optic sensor, or any other temperature sensor, or any combination of temperature sensors.

Although some examples have been provided for the type of detector 104, it will be understood that other types of detector, or combinations of detectors, are also possible. Returning back to Figure 1, the device 100 also comprises a user interface 106. The user interface 106 is for use in providing the user of the device 100 with information resulting from the method described herein. The processor 102 of the device 100 can be configured to control the user interface 106 to provide the information resulting from the method. The user interface 106 is, in particular, configured to indicate (or render, output, or provide) day-based information about the one or more monitored physiological characteristics of the user, wherein a start time point for the day-based information is determined based on the detected daily routine of the user. In some embodiments, an end time point for the day-based information is also determined based on the detected daily routine of the user. The end time point can be taken as the start time point for the day-based information according to some embodiments. In some embodiments, the user interface 106 is configured to indicate the day-based information about the monitored one or more physiological characteristics of the user, normalised to a 24 hour period.

In some embodiments, the day-based information about the one or more monitored physiological characteristics of the user may be cumulative or aggregated day-based information. Examples of day-based information include, but are not limited to, a count of a physiological characteristic (for example, a step count) for the day, an average of a physiological characteristic (for example, an average heart rate) for the day, a pattern of a physiological characteristic (for example, a movement pattern) for the day, a maximum value of a physiological characteristic (for example, a maximum heart rate value) for the day, a minimum value of a physiological characteristic (for example, a minimum heart rate value) for the day, or any other day-based information about the one or more monitored physiological characteristics of the user.

In some embodiments, in addition to providing the user of the device 100 with information resulting from the method described herein, the user interface 106 of the device 100 may also be configured to receive a user input. In other words, the user interface 106 may allow the user of the device 100 to manually enter data, instructions, or information. The processor 102 may be configured to acquire the user input from the user interface 106.

Thus, the user interface 106 of the device 100 may be any user interface that enables information resulting from the method described herein to be provided to the user of the device 100 and, optionally, can also be any user interface that enables the user of the device 100 to provide a user input, interact with and/or control the device 100. For example, the user interface 106 may comprise one or more switches, one or more buttons, a keypad, a keyboard, a mouse, a touch screen or an application (for example, on a smart device such as a tablet or smartphone), a display screen, a graphical user interface (GUI) or any other visual component, one or more speakers, one or more microphones or any other audio or acoustic component, one or more lights, a component for providing tactile feedback (such as a vibration function), or any other user interface components, or any combination of user interface components.

Returning again to Figure 1, in some embodiments, the device 100 may further comprise a communications interface (or circuitry) 108 for enabling the device 100 to communicate with (or connect to) any components, interfaces, units, memories, sensors and devices that are internal or external to the device 100. The communications interface 108 may communicate with any components, interfaces units, sensors and devices wirelessly or via a wired connection.

In embodiments where the processor 102 acquires information indicative of the period of sleep of the user of the device 100 from at least one separate device for the detection of the daily routine of the user (as mentioned earlier), the communications interface 108 may comprise a receiver that is configured to receive an input from the at least one separate device. For example, in some embodiments, the processor 102 may be configured to detect the daily routine of the user of the device by detecting one or more activities performed by the user of the device 100 and the receiver can, in this case, be configured to receive an input from the at least one separate device to detect the one or more activities performed by the user of the device 100.

As illustrated in Figure 1, in some embodiments, the device 100 may further comprise a memory 110 configured to store program code that can be executed by the processor 102 to perform the method described herein. Alternatively or in addition, one or more memories 110 may be external to (i.e. separate to or remote from) the device 100. For example, one or more memories 106 may be part of another device. A memory 110 can be used to store information resulting from the method described herein. Thus, for example, a memory 110 may be used to store the one or more monitored physiological characteristics of the user, the day-based information about the one or more monitored physiological characteristics of the user, or any other information resulting from the method described herein. The processor 102 can be configured to control a memory 110 to store the information resulting from the method disclosed herein.

It will be appreciated that Figure 1 only shows the components required to illustrate this aspect of the invention, and in a practical implementation the device 100 may comprise additional components to those shown. For example, the device 100 may comprise a battery or other power supply for powering the device 100 or means for connecting the device 100 to a mains power supply.

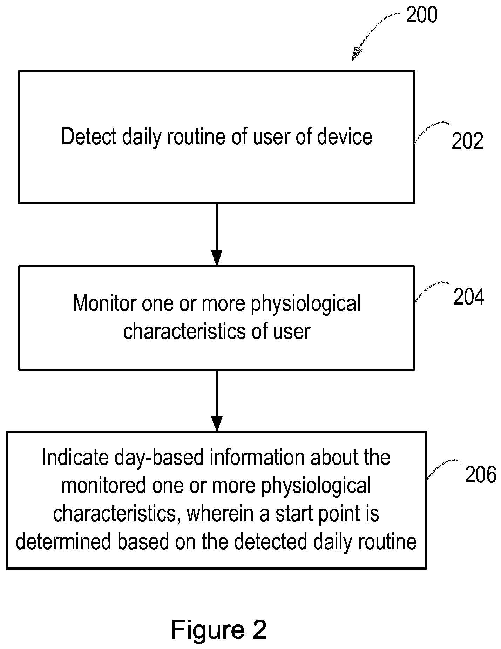

**Figure 2** illustrates a method 200 of operating a device for monitoring a user according to an embodiment. The illustrated method 200 can generally be performed by or under the control of the processor 102 of the device 100.

At block 202 of Figure 2, a daily routine (or daily pattern) of the user of the device 100 is detected. As mentioned earlier, the processor 102 of the device 100 is configured to detect a daily routine of the user of the device 100.

In some embodiments, the processor 102 may be configured to detect the daily routine of the user of the device 100 by detecting a period of sleep of the user of the device 100. In some embodiments, for example, the processor 102 may be configured to detect a period of sleep of the user of the device 100 exceeding a threshold duration. According to some embodiments, the threshold duration can be in the range of 5-6 hours or, preferably, in the range of 2-4 hours (for example, 3 hours). The threshold duration can be any threshold duration that is sufficiently short to ensure that a start time point of a day is detected even where the user of the device 100 sleeps for a minimum time period.

Alternatively or in addition, in some embodiments, the processor 102 may be configured to detect a period of sleep of the user of the device 100 based on a probability that the user is asleep (i.e. sleeping) or awake. In other words, a probabilistic implementation can be employed to detect a period of sleep of the user of the device 100. In these embodiments, information indicative of the period of sleep of the user acquired from one or more detectors 104 (such as a motion sensor, a heart rate sensor, or similar) may be compared to information indicative of a period of sleep of other users that is stored in a memory 110 with associated probabilities of the user being awake. For example, a certain level of physical activity detected by one or more detectors 104 may be stored with (or mapped to) a certain probability of the user being asleep. As previously mentioned, the memory 110 may be a memory of the device 100 or a memory external to (i.e. separate to or remote from) the device 100. A probabilistic implementation for detecting a period of sleep of the user can be particularly useful where the user has a sleeping disorder where it is not always reliable to detect the period of sleep of the user solely based on the length of time for which the user sleeps.

In any of the embodiments involving the detection of a period of sleep of the user, the processor 102 may be configured to exclude short periods of wakefulness when detecting the period of sleep.

Alternatively or in addition to the processor 102 being configured to detect the daily routine of the user of the device 100 by detecting a period of sleep of the user, the processor 102 may be configured to detect the daily routine of the user by detecting one or more activities performed by the user of the device 100. For example, in some embodiments, a receiver of the device 100 can be configured to receive an input from at least one separate device for the processor 102 to detect the one or more activities performed by the user of the device 100. As mentioned earlier, examples of activities that may be detected include, but are not limited to, movement patterns, time of meals, or any other activities, or any combination of activities. In some embodiments, the daily routine of the user can be detected by detecting one or more activities performed by the user of the device 100 (for example, after filtering out any activities unsuitable to the detection, such as night-time snacking), since the user can be defined as being awake if the one or more activities are being performed. Alternatively or in addition, a pattern of activities can be recognised compared to one or more previous days. This comparison can be used to determine the probability that the user is asleep at a certain time. For example, it may be recognised from one or more previous days that the user goes to sleep after a certain pattern of activities. Thus, when this recognised pattern of activities is next detected, it can be determined that the user goes to sleep afterwards since there is a high probability that they will in fact do so.

At block 204 of Figure 2, one or more physiological characteristics of the user are monitored. A detector 104 of the device 100 is configured to monitor the one or more physiological characteristics of the user. At block 206 of Figure 2, day-based information about the monitored one or more physiological characteristics of the user are indicated. The user interface 106 of the device 100 is configured to indicate the day-based information, wherein a start time point for the day-based information is determined based on the detected daily routine of the user.

For example, in embodiments where the processor 102 is configured to detect the daily routine of the user of the device 100 by detecting one or more activities performed by the user of the device 100, an occurrence of one or more activities associated with waking may be taken as the start time point for the day-based information. In some embodiments, the processor 102 may detect an occurrence of one or more activities associated with waking based on information received from one or more devices (which may be the device 100 itself, one or more separate devices, or a combination of both). For example, an occurrence of one or more activities associated with waking may comprise the user brushing their teeth, turning off an alarm clock, interacting with their phone, or any other activity associated with waking. In some embodiments, an occurrence of a pattern of activities may be used to determine a start time point for the day-based information. Similarly, in embodiments where the processor 102 is configured to detect the daily routine of the user of the device 100 by detecting a period of sleep of the user of the device 100, sleep detection itself may be used to detect the start time point for the day-based information. Specifically, a time point at which an end of a period of sleep of the user is detected can be determined as the start time point for the day-based information.

According to some embodiments, an end time point for the day-based information can be taken as the start time point for the day-based information. For example, in embodiments where the processor 102 is configured to detect the daily routine of the user of the device 100 by detecting a period of sleep of the user of the device 100, the end of the detected period of sleep may be taken as the start time point for the day-based information. In some embodiments, the user interface 106 is configured to indicate the day-based information about the monitored one or more physiological characteristics of the user, normalised to a 24 hour period.

Thus, according to the method described above, the observation window for the one or more monitored physiological characteristics of the user (or the interval to which a day corresponds) can be shifted, altered, adjusted, changed, or reset. In effect, the time interval for which information is indicated corresponds to the actual day of the user (or the day as it is perceived by the user). The actual day of the user can include a daytime period and a night-time period. For example, the actual day of the user can comprise a 24 hour representation of a day. For example, this time interval can correspond to the time interval between the time at which the user wakes up and the time at which the user goes to bed. In this way, the day-based information indicated via the user interface 106 provides the user with feedback on physiological characteristics that are associated with the day as the user actually perceives it.

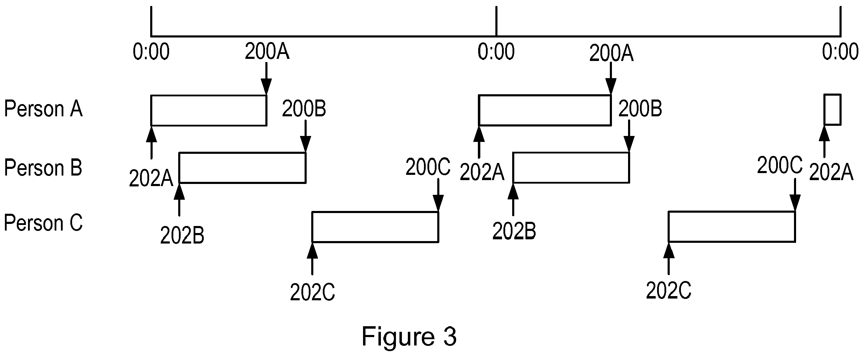

**Figure 3** illustrates an example of time intervals for day-based information determined according to an embodiment.

For person A, the start time points 200A for the day-based information are determined based on the detected daily routine of the user. The end time points 202A for the day-based information are the last points before the start of a new day at the subsequent start time point 200A. For person B, the start time points 200B for the day-based information are determined based on the detected daily routine of the user. The end time points 202B for the day-based information are the last points before the start of a new day at the subsequent start time point 200B. For person C, the start time points 200C for the day-based information are determined based on the detected daily routine of the user. The end time points 202C for the day-based information are the last points before the start of a new day at the subsequent start time point 200C. The start time points 200A, 200B and 200C and the end time points 202A, 202B and 202C correspond to those that are perceived by the user as being the start time points and end time points of a day.

In the illustrated example embodiment of Figure 3, person C may be a shift-worker. By employing the method described herein for person C, the day-based information comprises information acquired in the time interval between 200C and 202C, rather than only comprising the information acquired in the time interval between 0:00 and 202C (with the information from the time interval between 200C and 0:00 being omitted). To a lesser extent, the same can be seen to apply to Person B.

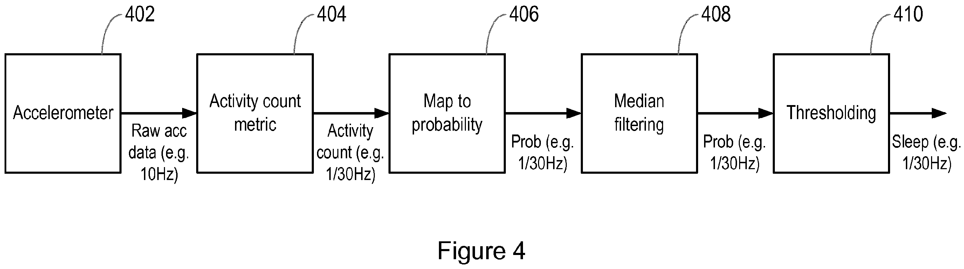

**Figure 4** illustrates a method of operating a device 100 for monitoring a user according to an example embodiment. The example embodiment employs sleep detection to detect the daily routine of the user of the device 100 and can generally be performed by or under the control of the processor 102 of the device 100. In this example embodiment, the device 100 is a wearable device comprising an accelerometer.

At block 404 of Figure 4, the accelerometer of the device 100 provides raw accelerometer data at a fixed sampling frequency (for example, at a sampling frequency of 10Hz) to the processor 102 of the device 100. The processor 102 accumulates the accelerometer data into a metric indicative of the amount of motion. Specifically, the accelerometer data is accumulated into an activity count metric. This may comprise accumulating a root mean square (RMS) of the unbiased accelerometer section in (for example, short) segments of data. The output is a signal at a fixed sampling frequency (for example, a sampling frequency of 1/30Hz or 1/60Hz).

At block 406 of Figure 4, the processor 102 of the device 100 maps each activity count to a corresponding pre-calculated probability that the user is sleeping for that time instance to acquire a probability curve. At block 408 of Figure 4, the processor 102 applies median filtering to smooth the probability curve. At block 410 of Figure 4, the processor 102 applies a threshold to the median probability curve. For example, according to some embodiments, a probability of less than 0.5 may be defined as the user being asleep, rather than awake. The processor 102 can then obtain a discrete state of "sleep" or "awake" for each time instance. In this way, the daily routine of the user is detected (as in block 202 of Figure 2).

One or more physiological characteristics of the user are monitored (as in block 204 of Figure 2) and thus, following the detection of the daily routine of the user, it is possible for the processor 102 to cause the user interface 106 to indicate day-based information about the one or more monitored physiological characteristics of the user (as in block 206 of Figure 2). In particular, according to the example embodiment of Figure 4, if the processor 102 determines that a portion of "sleep" is longer than a pre-defined threshold (for example, 3 hours), the processor 102 causes the user interface 106 to indicate the end of the sleep state as the start time point for the day-based information. In order to prevent small periods of wakefulness during sleep being indicated as the start of the day, additional processing may be included.

In the manner described above with reference to Figure 4, the end of sleep can essentially be used as a trigger to refresh (or restart) the day for the one or more monitored physiological characteristics. This can be advantageous where the one or more monitored physiological characteristics is a step count for the user in that a reset may already be carried out during a sufficiently long period of sleep, which can compensate for any latency that may be required for the detection of the end of sleep.

There is therefore provided an improved device and method of operating the device for monitoring a user. The method and device described herein can be useful in lifestyle monitoring programs or health programs, particularly those that use wearable devices and/or connected devices.

There is also provided a computer program product comprising a computer readable medium, the computer readable medium having computer readable code embodied therein, the computer readable code being configured such that, on execution by a suitable computer or processor, the computer or processor is caused to perform the method or methods described herein.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A device (100) for monitoring a user, the device (100) comprising:
a processor (102) configured to detect a daily routine of the user of the device;
a detector (104) configured to monitor one or more physiological characteristics of the user; and
a user interface (106) configured to indicate day-based information about the one or more monitored physiological characteristics of the user;
wherein a start time point for the day-based information is determined based on the detected daily routine of the user.

2. A device (100) as claimed in claim 1, wherein the processor (102) is configured to detect the daily routine of the user of the device by detecting a period of sleep of the user of the device (100).

3. A device (100) as claimed in claim 2, wherein the processor (102) is configured to detect a period of sleep of the user of the device exceeding a threshold duration.

4. A device (100) as claimed in claim 3, wherein the threshold duration is in the range of 2-4 hours.

5. A device (100) as claimed in any one of claims 2 to 4, wherein the processor (102) is configured to exclude short periods of wakefulness when detecting the period of sleep.

6. A device (100) as claimed in any one of claims 2 to 5, wherein the end of the detected period of sleep is taken as the start time point for the day-based information.

7. A device (100) as claimed in any one of claims 2 to 6, further comprising any one or more of:
a motion sensor, wherein the processor (102) is configured to detect the period of sleep of the user of the device from an output of the motion sensor; and
a heart rate sensor, wherein the processor (102) is configured to detect the period of sleep of the user of the device from an output of the heart rate sensor.

8. A device (100) as claimed in any one of the preceding claims, wherein the processor (102) is configured to detect a period of sleep of the user based on a probability that the user is asleep or awake.

9. A device (100) as claimed in claim 1, wherein the processor (102) is configured to detect the daily routine of the user of the device by detecting any one or more of:
one or more activities performed by the user of the device (100); and
an interaction of the user with the device (100) or another device.

10. A device (100) as claimed in claim 9, wherein an occurrence of one or more activities associated with waking is taken as the start time point for the day-based information.

11. A device (100) as claimed in any one of claims 9 or 10, further comprising:
a receiver configured to receive an input from at least one separate device to detect the one or more activities performed by the user of the device (100).

12. A device (100) as claimed in any one of claims 1 to 11, wherein the detector (104) comprises any one or more of:
a motion sensor;
a heart rate sensor;
a respiratory rate sensor;
a skin response sensor;
a muscle activity sensor; and
a temperature sensor.

13. A device (100) as claimed in any one of claims 1 to 12, wherein the user interface (106) is configured to indicate the day-based information about the one or more monitored physiological characteristics of the user, normalised to a 24 hour period.

14. A method (200) of operating a device (100) for monitoring a user, the method comprising:
detecting (202) a daily routine of the user of the device (100);
monitoring (204) one or more physiological characteristics of the user; and
indicating (206) day-based information about the one or more monitored physiological characteristics of the user;
wherein a start time point for the day-based information is determined based on the detected daily routine of the user.

15. A computer program product comprising a computer readable medium, the computer readable medium having computer readable code embodied therein, the computer readable code being configured such that, on execution by a suitable computer or processor, the computer or processor is caused to perform the method of claim 14.
